# EUROPEAN PATENT APPLICATION

(11) **EP 2 116 525 A1**
(43) Date of publication of application: **11.11.2009**
(21) Application number: 09159342.6
(22) Date of filing: 04.05.2009
(51) Int. Cl.: C07C 231/24, C07C 237/26, A61K 31/165

(54) **Process for the preparation of tigecycline in the amorphous form**

(30) Priority: 05.05.2008 IT MI20080807
(71) Applicant: Antibioticos S.p.A., 20090 Rodano (MI) (IT)
(72) Inventor: Pozzi, Giovanni, 20090 Rodano (MI) (IT); Alpegiani, Marco, 20090 Rodano (MI) (IT); Bedeschi, Angelo, 20090 Rodano (MI) (IT); Pizzocaro, Roberta, 20090 Rodano (MI) (IT)
(74) Representative: Minoja, Fabrizio

(57) **Abstract**

The invention relates to processes for the preparation of tigecycline (**I**): 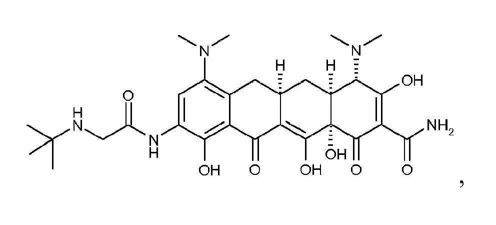 a wide spectrum antibiotic belonging to the tetracycline family, in stable amorphous form, by freeze-drying, antisolvent precipitation and nebulization.

## Description

### Field of the Invention

The present invention relates to processes for the preparation of the tigecycline (**I**) in stable amorphous form.

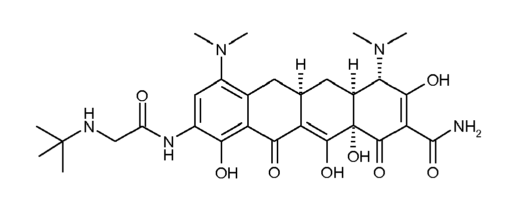

### Technological Background

Tigecycline is a glycylcycline belonging to the tetracycline family, structurally related to minocycline. It is a wide spectrum antibiotic, active against Gram-positive and Gram-negative bacteria, including tetracycline-resistant microorganisms, and against some anaerobics.

The family of products comprising tigecycline is disclosed in US 5494903 and in corresponding EP 536515; processes for its preparation are described, for example, in US 2007/0026080. US 2007/0026081, US 2007/0026082 and US 2007/0026083; crystalline forms (polymorphs) are disclosed, for example, in US 2007/0123497.

Tigecycline is at present prepared on an industrial scale in the form of a lyophilized powder for the intravenous use, for reconstitution in water for injection prior to use. The typical process for the preparation of the lyophilized powder comprises dissolving tigecycline in water and freeze-drying the aqueous solution to obtain an amorphous solid. Freeze-drying from water is however rather problematic to carry out on an industrial scale, as the oxygen contained in water induces degradation of tigecycline to give oxidation and epimerization products in pharmaceutically unacceptable amounts. In order to obviate to this drawback, US 2007/0026080 suggests a freeze-drying process in which the water for injection used to prepare the starting solution is subjected to the substantial removal of the oxygen and is brought to a temperature ranging from 2 to 8°C before adding tigecycline and freeze-drying the solution.

An alternative process which provides amorphous tigecycline with low impurity content and does not require the preventive deoxygenation of the starting solution, would therefore be advantageous from the industrial point of view.

### Disclosure of the invention

The present invention relates to processes for the preparation of tigecycline in amorphous form which do not involve freeze-drying of aqueous solutions. All of said processes comprise the precipitation of tigecycline from a solution prepared dissolving tigecycline, obtained according to methods known in literature, in a suitable solvent, to attain a concentration at least higher than 5% w/w, preferably higher than 10% w/w.

In a first aspect, the invention relates to a process for the preparation of tigecycline in amorphous form, comprising freeze-drying a tigecycline solution in an anhydrous organic solvent or in a mixture of two or more anhydrous organic solvents, in which the term "anhydrous" means a water content lower than 1%, preferably lower than 0.5%. The term "organic solvent" means a solvent selected from dioxane, tert-butanol, dimethyl carbonate or mixtures thereof, or mixtures with other solvents, such as methanol, ethanol and acetonitrile. The freeze-drying process is effected with methods well known to those skilled in the art. The tigecycline solution is cooled to a temperature lower than the solidification one and drying is carried out *in vacuo* at temperatures which allow sublimation of the solvent (or solvent mixture). The operative pressure is usually in the range of 10 to 500 µbars, and the temperature is adjusted to control the sublimation rate of the solvent and to avoid mass melting phenomena. The sublimation rate is monitored by measuring the residual vacuum which should be kept within the range as defined above. The operative temperature usually falls in the range of -70°C to +70°C.

In a second aspect, the invention relates to a process for the preparation of tigecycline in amorphous form comprising adding an antisolvent to a tigecycline solution. The tigecycline solution is prepared using an organic solvent selected from alcohols, e.g. ethanol, methanol or iso-propanol; ketones, e.g. acetone or methyl-ethyl-ketone; amides, e.g. N,N-dimethylformamide, N,N-dimethylacetamide or N-methylpyrrolidone; ethers, e.g. tetrahydrofuran, methyl-tetrahydrofuran or dioxane; esters, e.g. methyl acetate, methyl formate or ethyl acetate; nitriles, e.g. acetonitrile; carbonates, e.g. dimethyl carbonate or ethylene carbonate; sulfoxides, e.g. dimethylsulfoxide; halo solvents, e.g. methylene chloride; or mixtures consisting of two or more of these solvents. In this case, mixtures with water can also be used, as far as the water content is lower than 5%. Once obtained an homogeneous solution, either the anti-solvent is added, or the tigecycline solution is poured into the anti-solvent. An amount of anti-solvent ranging from 1 to 20 volumes on the volume of the tigecycline solution is usually employed.

Anti-solvents comprise low polarity organic solvents, preferably those miscible with the solvent, for example alkanes, e.g. heptane, hexane or butyl chloride; aromatic hydrocarbons, e.g. xylene or toluene; ethers, e.g. tert-butyl-methyl ether, iso-propyl ether or cyclopentyl-methyl ether; esters, e.g. propyl acetate or butyl acetate, or mixtures thereof. The resulting precipitate is an amorphous solid which is then recovered by known techniques, for example by filtration, centrifugation or decantation. After suitable washing to remove the impurities, the humid solid is dried at a pressure of 1 mbar to 1 atmosphere and at a temperature in the range from 10°C to 80°C, preferably from 15°C to 70°C.

In a third aspect, tigecycline in amorphous form is prepared by spray-drying, which comprises nebulization of a tigecycline solution, drying in gas stream and recovering the product. When using this technique, tigecycline can be dissolved in water alone, or in the same solvents as used with the antisolvent precipitation technique, optionally in mixture with water; preferred solvents are dichloromethane, ethanol, methanol, tetrahydrofuran, water. In this case also, mixtures of two or more of said solvents can be used. For example, methanol-dichloromethane, tetrahydrofuran-ethanol and ethanol-water mixtures can be used. The drying gas is typically an inert gas such as nitrogen or carbon dioxide, and the gas temperature can range from 0°C to 150°C, preferably from 20°C to 130°C.

The invention will be now illustrated in more detail by the following examples.

### EXAMPLES

### Example 1. Freeze-drying

A solution of tigecycline (1.5 g) in dioxane (30 ml) is cooled to -30°C. The freeze-drying tray is connected to a high vacuum pump, and temperature is set first at -30°C, after 3 hours at -20°C, after a further 3 hours at -5°C and finally at +5°C to complete solvent sublimation. 1.5 g of an amorphous product is obtained (analysis at the polarized light optical microscope).

### Example 2. Spray-drying

Crude tigecycline is treated with active charcoal in a dichloromethane and methanol 95:5 mixture. The mixture is stirred at room temperature for 1 hour, then filtered and the filtrate is dried by spray-drying under nitrogen stream (inlet temperature 60-70°C, outlet temperature 30-40°C). An amorphous product is obtained.

### Example 3. Spray-drying

A crude tigecycline solution (2.0 g) in water (10 ml) is dried by means of spray-drying in nitrogen stream (inlet temperature 100-120°C, outlet temperature 70-80°C). An amorphous product is obtained.

### Example 4. Anti-solvent precipitation

A solution of crude tigecycline (2.0 g) in tetrahydrofuran (20 ml) is slowly added to a solution of tert-butyl methyl ether (100 ml) and cooled to -10°C under strong stirring using an Ultra-Turrax. The solution is filtered and dried under vacuum, thereby obtaining an amorphous product.

## Claims

1. A process for the preparation tigecycline in stable amorphous form, which comprises freeze-drying a tigecycline solution having a concentration not lower than 5% (w/w) in an anhydrous organic solvent selected from dioxane, tert-butanol, dimethyl carbonate or mixtures thereof, or mixtures thereof with methanol, ethanol and acetonitrile, at an operative pressure ranging from 10 µbar to 500 µbar and at a temperature ranging from -70°C to +70°C.

2. A process for the preparation tigecycline in the amorphous form, which comprises preparing a tigecycline solution in a solvent selected from ethanol, methanol, isopropanol, acetone, methyl-ethyl-ketone, N,N-dimethylformamide, N,N-dimethylacetamide, N-methylpyrrolidone, tetrahydrofuran, methyl-tetrahydrofuran, dioxane, methyl acetate, methyl formate, ethyl acetate, acetonitrile, dimethyl carbonate, ethylene carbonate, dimethylsulfoxide, methylene chloride or mixtures consisting of two or more of said solvents, and adding an anti-solvent to said solution, or adding said solution to an anti-solvent selected from heptane, hexane, butyl chloride, xylene, toluene, tert-butyl-methyl ether, iso-propyl ether, cyclopentyl-methyl ether, propyl acetate, butyl acetate, or mixtures thereof, in which the amount of anti-solvent ranges from 1 to 20 volumes based on the tigecycline solution volume.

3. A process the preparation of tigecycline in amorphous form, which comprises nebulizing a tigecycline solution having a concentration not lower than 5% (w/w) in an anhydrous organic solvent selected from dichloromethane, ethanol, methanol, tetrahydrofuran, water or mixtures thereof, and drying in nitrogen or carbon dioxide stream at a temperature ranging from 0°C to 150°C, preferably from 20°C to 130°C.

4. Tigecycline in the amorphous form obtained according to the process of any one of claims 1 to 3.
